# EUROPÄISCHE PATENTSCHRIFT

(11) **EP 4 167 996 B1**
(45) Veröffentlichungstag und Bekanntmachung des Hinweises auf die Patenterteilung: **01.05.2024**
(21) Anmeldenummer: 21725751.8
(22) Anmeldetag: 12.05.2021
(51) Int. Cl.: A61K 31/4152, A61P 9/12

(54) **METAMIZOLDERIVATE ZUR PRÄVENTION UND THERAPIE DER PULMONALEN HYPERTONIE**
METAMIZOLE DERIVATIVES FOR THE PREVENTION AND TREATMENT OF PULMONARY HYPERTENSION
DÉRIVÉS DE MÉTAMIZOLE POUR LA PRÉVENTION ET LE TRAITEMENT DE L'HYPERTENSION PULMONAIRE

(30) Priorität: 18.06.2020 DE 102020116104
(43) Veröffentlichungstag der Anmeldung: 26.04.2023
(73) Patentinhaber: Preißner, Robert, 10407 Berlin (DE); Preißner, Saskia, 10249 Berlin (DE); Gohlke, Björn-Oliver, 12279 Berlin (DE); Kübler, Wolfgang, 10965 Berlin (DE)
(72) Erfinder: PREISSNER, Robert, 10407 Berlin (DE); GOHLKE, Björn-Oliver, 12279 Berlin (DE); PREISSNER, Saskia, 10249 Berlin (DE); KÜBLER, Wolfgang, 10965 Berlin (DE); GRIMMER, Benjamin, 10407 Berlin (DE)
(74) Vertreter: Zimmermann & Partner Patentanwälte mbB
(86) Internationale Anmeldenummer: PCT/EP2021/062639
(87) Internationale Veröffentlichungsnummer: WO 2021/254697

(56) Entgegenhaltungen:
- GB-A- 2 344 995
- ZO RAKOTONIAINA ET AL: "Celecoxib but not the combination of celecoxib+atorvastatin prevents the development of monocrotaline-induced pulmonary hypertension in the rat", NAUNYN-SCHMIEDEBERG'S ARCHIVES OF PHARMACOLOGY, SPRINGER, BERLIN, DE, Bd. 378, Nr. 3, 10. Juni 2008 (2008-06-10) , Seiten 241-251, XP019620950, ISSN: 1432-1912
- SEHGAL ARVIND ET AL: "Pulmonary Hypertension in an Infant Treated with Ibuprofen", INDIAN JOURNAL OF PEDIATRICS., Bd. 80, Nr. 8, 1. August 2013 (2013-08-01) , Seiten 697-699, XP055820235, IN ISSN: 0019-5456, DOI: 10.1007/s12098-012-0829-2 Gefunden im Internet: URL:https://link.springer.com/content/pdf/ 10.1007/s12098-012-0829-2.pdf>
- HERTEL MORITZ ET AL: "Real-world evidence for beneficial effects of dipyrone in 4,278 patients with pulmonary hypertension - Analysis of the risk of ventilation, hospitalization, and agranulocytosis", EUROPEAN JOURNAL OF PHARMACOLOGY, ELSEVIER SCIENCE, NL, vol. 943, 6 February 2023 (2023-02-06), ISSN: 0014-2999, DOI: 10.1016/J.EJPHAR.2023.175567 [retrieved on 2023-02-06]

## Beschreibung

Die Erfindung betrifft einen medizinischen Wirkstoff gemäß den Ansprüchen, dessen pharmazeutisch geeignete Salze, deren Solvate zur Verwendung in der therapeutischen Behandlung und/oder Prävention der pulmonalen Hypertonie.

### Technologischer Hintergrund

Das kennzeichnende Merkmal einer pulmonalen Hypertonie (PH) ist ein erhöhter Blutdruck in den Lungenarterien. Dies kann auf Dauer zu einer Belastung des Herzmuskels führen, wodurch insbesondere die rechte Herzkammer sich mit zunehmender Dauer weitet und an Kraft verliert, so dass sie letztlich die notwendige Blutmenge nicht mehr transportieren kann. Typische Anzeichen der pulmonalen Hypertonie sind verengte Lungenarterien mit einer verdickten Gefäßwand.

Als direkte Folge ist die Sauerstoffversorgung des Körpers gemindert und die Leistungsfähigkeit der Betroffenen drastisch einschränkt. Im fortgeschrittenen Stadium kann sich die pulmonale Hypertonie zu einem lebensbedrohlichen Zustand entwickeln.

Die Diagnose einer idiopathischen pulmonalen Hypertonie erfolgt sehr selten. Deutlich häufiger ist die PH die Folge einer Vorerkrankung, beispielsweise einer Erkrankung des linken Herzens oder von Lungenkrankheiten und/oder Sauerstoffmangel (Hypoxie). Zu den Symptomen der PH zählen Luftnot, Schwäche, schwere Kreislaufstörungen und schließlich Rechtsherzinsuffizienz. Patienten, die an PH erkranken, versterben zumeist an einer rechtskardialen Dekompensation.

Für die Therapie der pulmonalen Hypertonie sind derzeit nur wenige Medikamente zugelassen. Zu den Wirkstoffen zählen Prostanoide, sGC-Stimulatoren (Riociguat), Endothelin-Rezeptorantagonisten und Phosphodiesterase-5-Inhibitoren. Die Effektivität der Wirkstoffe ist jedoch gering und die Medikationen führen in der Regel nicht zu einer Heilung, sondern verzögern nur das Fortschreiten des Krankheitsprozesses. Zudem können erhebliche Nebenwirkungen auftreten, sodass mitunter statt der medikamentösen Therapie eine Lungentransplantation von Nöten ist. RAKOTONIAINA et al.; NAUNYN-SCHMIEDEBERG'S ARCHIVES OF PHARMACOLOGY, Bd. 378, Nr. 3, 2008, S. 241-251 offenbart Celecoxib zur Behandlung der pulmonalen Hypertonie.

Die Zielsetzung der vorliegenden Erfindung ist demnach die Bereitstellung eines effektiven medizinischen Wirkstoffs zur Therapie und Prävention von pulmonaler Hypertonie.

### Zusammenfassung der Erfindung

Der Gegenstand der vorliegenden Erfindung ist ein medizinischer Wirkstoff gemäß Formel (I): 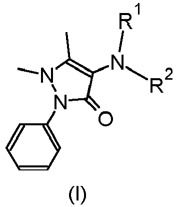 dessen pharmazeutisch geeignete Salze und Solvate, Solvate der pharmazeutisch geeigneten Salze des genannten Wirkstoffs zur Verwendung in der therapeutischen Behandlung und/oder Prävention von pulmonaler Hypertonie.

In Formel (I) entsprechen R¹ und R² unabhängig voneinander ausgewählten Substituenten aus der Gruppe umfassend Wasserstoff, substituierte und unsubstituierte C1-C4 Alkylgruppen, wobei bevorzugt die substituierte C1-C4 Alkylgruppe über 1 bis 4 Kohlenstoffatome verfügt und deren Wasserstoffatome einfach oder mehrfach durch Deuterium oder Fluor oder -SO₃H substituiert sind.

Der Begriff "Pulmonale Hypertonie" (PH) kann hierbei die pulmonalarterielle Hypertonie (PAH), die pulmonale Hypertonie bei Erkrankungen des linken Herzens, die pulmonale Hypertonie bei Lungenerkrankungen und/oder Hypoxie, und die pulmonale Hypertonie bei chronischer Thromboembolieumfassen.

Unter "substituierte C1-C4 Alkylgruppe" wird in der zuvor genannten Formel (I) eine Alkylgruppe verstanden, welche über 1 bis 4 Kohlenstoffatome verfügt und deren Wasserstoffatome einfach oder mehrfach durch Deuterium oder Fluor oder -SO₃H substituiert sind. Vorzugsweise können "substituierte C1-C4 Alkylgruppen" aus der Gruppe umfassend: - CD₃, -CH₂CF₃ und -CH₂SO₃H ausgewählt sein. Bevorzugt kann die "substituierte C1-C4 Alkylgruppe" -CD₃ oder -CH₂SO₃H entsprechen. Besonders bevorzugt kann die "substituierte C1-C4 Alkylgruppe" -CH₂SO₃H entsprechen.

Unter "unsubstituierte C1-C4 Alkylgruppe" wird in der oben genannten Formel (I) eine verzweigte oder unverzweigte Alkylgruppe verstanden, welche über 1 bis 4 Kohlenstoffatome verfügt. Vorzugsweise können "unsubstituierte C1-C4 Alkylgruppen" aus der Gruppe umfassend: Methyl, Ethyl, *n*-Propyl, *n*-Butyl, *iso*-Propyl, *tert*-Butyl, und Butan-2-yl ausgewählt sein. Besonders bevorzugt kann die "unsubstituierte C1-C4 Alkylgruppe" einer Methylgruppe (bzw. -CH₃) entsprechen.

Unter "Solvat" wird ein Komplex aus einer Verbindung gemäß Formel (I) mit einem oder mehreren Lösungsmittelmolekülen verstanden. Ferner wird unter "Solvat" ein Komplex aus einem pharmazeutisch geeigneten Salz einer Verbindung gemäß Formel (I) mit einem oder mehreren Lösungsmittelmolekülen verstanden. Bevorzugt kann das Solvat einem Hydrat (z.B. ein Monohydrat) entsprechen. Das heißt, die Erfindung umfasst Solvate des Wirkstoffs gemäß Formel (I) und Solvate der pharmazeutisch geeigneten Salze des genannten Wirkstoffs zur therapeutischen Behandlung und/oder Prävention von pulmonaler Hypertonie.

Bevorzugte Ausführungsformen des medizinischen Wirkstoffs gemäß Formel (I) zur Verwendung in der therapeutischen Behandlung und/oder Prävention von pulmonaler Hypertonie können Verbindungen ausgewählt aus der Gruppe umfassend: 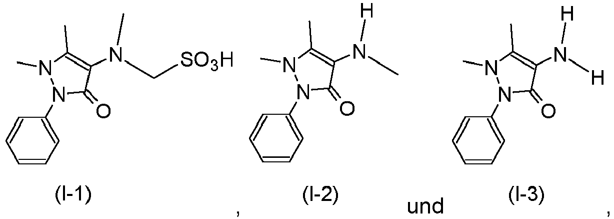 und deren pharmazeutisch geeignete Salze und Solvate umfassen. Besondere Ausführungsformen können demnach 4-Methylaminoantipyrin (I-2) und 4-Aminoantipyrin (I-3) sowie deren pharmazeutisch geeignete Salze und Solvate umfassen. Eine weitere bevorzugte Ausführungsform des medizinischen Wirkstoffs zur genannten medizinischen Verwendung ist das Monohydrat des Natriumsalzes der Verbindung (I-1) (auch Natrium[(1,5-Dimethyl-3-oxo-2-phenylpyrazol-4-yl)-methylamino]methansulfonat monohydrat; CAS: 5907-38-0).

Pharmazeutisch geeignete Salze des medizinischen Wirkstoffs können bevorzugt ein Kation umfassen, welches aus der Gruppe umfassend: Natrium, Kalium, Calcium und Magnesium ausgewählt ist. Ein Ausführungsbeispiel eines pharmazeutisch geeigneten Salz des medizinischen Wirkstoffs ist Natrium[(1,5-Dimethyl-3-oxo-2-phenylpyrazol-4-yl)-methylamino]methansulfonat (CAS: 68-89-3).

Ferner können pharmazeutisch geeignete Salze ein Anion umfassen, das aus der Gruppe umfassend: Acetat, Lactat, Citrat, Oxalat, Glutarat, Malat, Tartrat, Bitartrat, Fumarat, Mandelat, Maleat, Succinat, Benzoat, Chlorid, Hydrogencarbonat und Phthalat ausgewählt ist. Entsprechende pharmazeutisch geeignete Salze lassen sich über eine Protonierung des Wirkstoffs durch die korrespondierenden Säuren (beispielsweise Essigsäure zur Herstellung des Acetat-Salzes) oder durch lonenaustauschreaktionen (beispielsweise mittels Ionenaustauscher-Harzen) herstellen. Pharmazeutisch geeignete Salze die über eines der genannten Anionen verfügen können die Löslichkeit und die pH-abhängige Bioverfügbarkeit am Wirkort des Wirkstoffs steigern.

Ein weiterer Aspekt der Erfindung betrifft eine pharmazeutische Formulierung zur Verwendung in der therapeutischen Behandlung und/oder Prävention von pulmonaler Hypertonie, welche den medizinischen Wirkstoff wie zuvor definiert und einen pharmazeutisch geeigneten Hilfsstoff umfasst.

Der pharmazeutisch geeignete Hilfsstoff ist bevorzugt pharmakologisch inert und nicht-toxisch.

Der pharmazeutisch geeignete Hilfsstoff kann vorzugsweise aus der Gruppe umfassend: Lactose, Cellulose, Stärke, Saccharose, Paraffin, Hartfett, Polyethylengylkol (PEG), Polyethylenoxid (PEO), Wasser, Ethanol, Elektrolytlösung ausgewählt sein.

Die pharmazeutische Formulierung kann den medizinischen Wirkstoff in einer Dosierung von 10 bis 500 mg/kg Körpergewicht, vorzugsweise 50 bis 200 mg/kg Körpergewicht, besonders bevorzugt 70 bis 80 mg/kg Körpergewicht enthalten. Die Einheit "mg/kg Körpergewicht" wird hierin verstanden als Milligramm des zu verabreichenden Wirkstoffs pro Kilogramm des Körpergewichts des Behandelten.

Die pharmazeutische Formulierung kann bevorzugt in einer Darreichungsform umfassend Tabletten, Brausetabletten, Pillen, Hartkapseln, Weichkapseln, Zäpfchen, Injektionslösungen, Pulver, Granulat, Inhalationsspray, Spray und Salben bereitgestellt werden. Besonders bevorzugt kann die Darreichungsform eines Inhalationsspray oder einer Injektionslösung gewählt sein, da diese zu einer guten Bioverfügbarkeit am Wirkort beitragen.

### Kurzbeschreibung der Figuren

Die Erfindung wird nachfolgend anhand eines Ausführungsbeispiels und dazugehöriger Figuren näher erläutert. Die Figuren zeigen:
Fig. 1 Einfluss des erfindungsgemäßen Wirkstoffs auf die hypoxische pulmonale Vasokonstriktion.
Fig. 2 Einfluss des erfindungsgemäßen Wirkstoffs auf die hypoxische pulmonale Vasokonstriktion in Abhängigkeit der Konzentration.

### Detaillierte Beschreibung der Erfindung

### Versuchsdurchführung

### Hypoxisch pulmonale Vasokonstriktion an der isoliert-perfundierten Mauslunqe (IPL)

Für die IPL wurden 10-12 Wochen alte C57BU6J Mäuse verwendet. Zu Beginn wurden die Mäuse anästhesiert (Ketamin 100mg/kg Körpergewicht (KG); Xylazin 20mg/kg KG) und mittels zervikaler Dislokation getötet. Im Anschluss wurde eine Tracheotomie durchgeführt und die Lunge *in situ* positiv mit normoxischem (20% O₂; 5% CO₂; 75% N₂) Gas ventiliert (Tidalvolumen: 100 µl; Atemzüge pro Minute: Körpergewicht x 10). Anschließend wurde der Bauchraum eröffnet, das Diaphragma durchtrennt und Heparin (100 U) in den rechten Ventrikel injiziert. Nach der Eröffnung des Thorax mittels Sternotomie wurde sowohl die Pulmonalarterie als auch der linke Ventrikel kanüliert und die Lunge mit auf 37°C temperierter, gepufferter Salzlösung nach Hanks, angereichert mit Ca²⁺, Mg²⁺ und 10% FBS, perfundiert. Je nach Versuchsgruppe wurde das Perfusat zusätzlich mit einer Gewichts-entsprechenden Menge an Metamizol, 4-Aminoantipyrin oder 4-Methylamioantipyrin versetzt. Sobald sich der pulmonalarterielle Druck (PAP) stabilisiert hatte, wurden die Basalwerte aufgezeichnet und anschließend die Mauslunge mit hypoxischem Gas (1% O₂; 5% CO₂; 94% N₂) ventiliert. Veränderungen des PAP wurden mithilfe eines Drucksensors P75 (Havartus apparatus) detektiert, und geben das Ausmaß der hypoxischen Vasokonstriktion der Lunge wieder.

### Ergebnisse

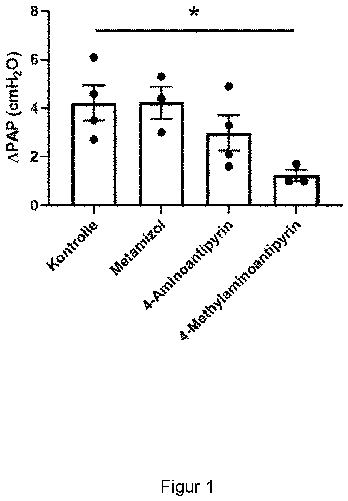

Die Ergebnisse des Einflusses des erfindungsgemäßen Wirkstoffs auf die hypoxische pulmonale Vasokonstriktion sind in Figur 1 dargestellt. Es wurden isoliert-perfundierte Mauslungen entweder mit gepufferter Salzlösung nach Hanks ohne Zusatz (Kontrolle) oder nach Zusatz von Metamizol, 4-Aminoantipyrin oder 4-Methylaminoantipyrin (200 mg/kg KG) perfundiert. Nach Umschaltung der Beatmung von normoxischem auf hypoxisches Gas (1% O₂; 5% CO₂; 94% N₂) stieg der pulmonalarterielle Druck in der Kontrollgruppe um ca. 4 cmH₂O an (ΔPAP). Die Behandlung der Lunge mit 4-Methylaminoantipyrin reduzierte den ΔPAP nach Umschalten auf Hypoxie um 70%. Mittelwert + SEM. P<0,005. Somit eignen sich die untersuchten Wirkstoffe für die Behandlung und Prävention der pulmonalen Hypertonie.

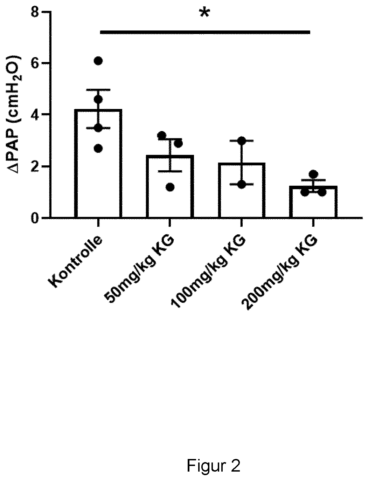

Figur 2 zeigt den Einfluss des erfindungsgemäßen Wirkstoffs auf die hypoxische pulmonale Vasokonstriktion in Abhängigkeit der Konzentration. Hierzu wurden isoliert-perfundierte Mauslungen entweder mit gepufferter Salzlösung nach Hanks ohne Zusatz (Kontrolle) oder nach Zusatz von 4-Methylaminoantipyrin (4-MAA) in unterschiedlicher Konzentration (50-200 mg/kg KG) perfundiert. Nach Umschaltung der Beatmung von normoxischem auf hypoxisches Gas (1% O₂; 5% CO₂; 94% N₂) stieg der pulmonalarterielle Druck in der Kontrollgruppe um ca. 4 cmH₂O an (ΔPAP). Die Behandlung der Lunge mit 4-MAA führte zu einer Dosis-abhängigen Reduktion des ΔPAP. Mittelwert + SEM. P<0,005. Somit eignet sich der untersuchte Wirkstoff insbesondere in den untersuchten Darreichungskonzentrationen für die Behandlung und Prävention der pulmonalen Hypertonie.

## Patentansprüche

1. Ein medizinischer Wirkstoff gemäß Formel (I): 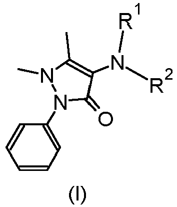
dessen pharmazeutisch geeignete Salze und Solvate, Solvate der pharmazeutisch geeigneten Salze des genannten Wirkstoffs zur Verwendung in der therapeutischen Behandlung und/oder Prävention von pulmonaler Hypertonie,
wobei R¹ und R² unabhängig voneinander aus der Gruppe umfassend Wasserstoff, substituierte und unsubstituierte C1-C4 Alkylgruppe ausgewählt sind, und
wobei die substituierte C1-C4 Alkylgruppe über 1 bis 4 Kohlenstoffatome verfügt und deren Wasserstoffatome einfach oder mehrfach durch Deuterium oder Fluor oder - SO₃H substituiert sind.

2. Der medizinische Wirkstoff zur Verwendung in der therapeutischen Behandlung und/oder Prävention von
pulmonaler Hypertonie nach Anspruch 1, wobei der medizinische Wirkstoff eine Verbindung ausgewählt aus der Gruppe umfassend: 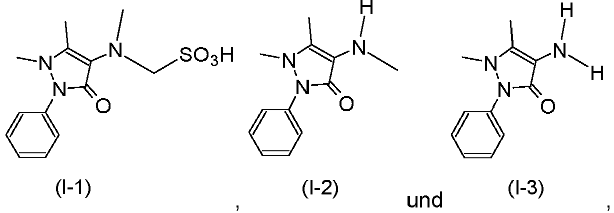
und deren pharmazeutisch geeignete Salze, Solvate umfasst.

3. Der medizinische Wirkstoff zur Verwendung in der therapeutischen Behandlung und/oder Prävention von pulmonaler Hypertonie nach Anspruch 1 oder 2, wobei die pulmonale Hypertonie eine pulmonal-arterielle Hypertonie, eine pulmonale Hypertonie bei Erkrankungen des linken Herzens, eine pulmonale Hypertonie bei Lungenerkrankungen und/oder Hypoxie, und eine pulmonale Hypertonie bei chronischer Thromboembolie umfasst.

4. Der medizinische Wirkstoff zur Verwendung nach einem der vorhergehenden Ansprüche, wobei das Solvat ein Hydrat ist.

5. Der medizinische Wirkstoff zur Verwendung nach einem der vorhergehenden Ansprüche, wobei das pharmazeutisch geeignete Salz ein Kation umfasst, das aus der Gruppe umfassend: Natrium, Kalium, Calcium und Magnesium ausgewählt ist.

6. Der medizinische Wirkstoff zur Verwendung nach einem der Ansprüche 1 bis 4, wobei das pharmazeutisch geeignete Salz ein Anion umfasst, das aus der Gruppe umfassend: Acetat, Lactat, Citrat, Oxalat, Glutarat, Malat, Tartrat, Bitartrat, Fumarat, Mandelat, Maleat, Succinat, Benzoat, Chlorid, Hydrogencarbonat und Phthalat ausgewählt ist.

7. Eine pharmazeutische Formulierung zur Verwendung in der therapeutischen Behandlung und/oder Prävention von pulmonaler Hypertonie, umfassend den medizinischen Wirkstoff nach einem der vorhergehenden Ansprüche und einen pharmazeutisch geeigneten Hilfsstoff.

8. Die pharmazeutische Formulierung zur Verwendung in der therapeutischen Behandlung und/oder Prävention nach Anspruch 7, wobei der pharmazeutisch geeignete Hilfsstoff aus der Gruppe umfassend Lactose, Cellulose, Stärke, Saccharose, Paraffin, Hartfett, Polyethylengylkol (PEG), Polyethylenoxid (PEO), Wasser, Ethanol, Elektrolytlösung ausgewählt ist.

9. Die pharmazeutische Formulierung zur Verwendung in der therapeutischen Behandlung und/oder Prävention nach Anspruch 7 oder 8, wobei die pharmazeutische Formulierung den medizinische Wirkstoff in einer Dosierung von 10 bis 500 mg/kg Körpergewicht, vorzugsweise 50 bis 200 mg/kg Körpergewicht, besonders bevorzugt 70 bis 80 mg/kg Körpergewicht umfasst.

10. Die pharmazeutische Formulierung zur Verwendung in der therapeutischen Behandlung und/oder Prävention nach einem der Ansprüche 7 bis 9, wobei die pharmazeutische Formulierung in einer Darreichungsform ausgewählt aus der Gruppe umfassend Tabletten, Brausetabletten, Pillen, Hartkapseln, Weichkapseln, Zäpfchen, Injektionslösungen, Pulver, Granulat, Inhalationsspray, Spray und Salben bereitgestellt wird.

## Claims

1. A medical active ingredient according to formula (I): 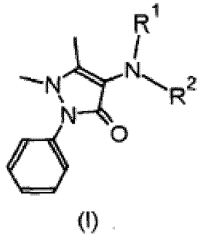
pharmaceutically acceptable salts and solvates thereof, solvates of the pharmaceutically acceptable salts of the active ingredient for use in the therapeutic treatment and/or prevention of pulmonary hypertension,
wherein R¹ and R² are independently selected from the group consisting of hydrogen, substituted and unsubstituted C1-C4 alkyl group, and
wherein the substituted C1-C4 alkyl group has 1 to 4 carbon atoms and its hydrogen atoms are mono- or polysubstituted by deuterium or fluorine or SO₃H.

2. The medical active ingredient for use in the therapeutic treatment and/or prevention of pulmonary hypertension according to claim 1, wherein the medical active ingredient comprises a compound selected from the group consisting of: 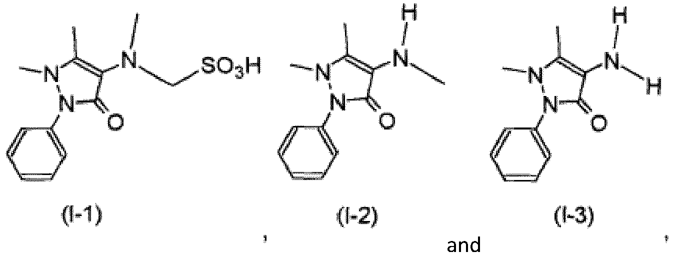 and pharmaceutically acceptable salts, solvates thereof.

3. The medical active ingredient for use in the therapeutic treatment and/or prevention of pulmonary hypertension according to claim 1 or 2, wherein the pulmonary hypertension comprises pulmonary arterial hypertension, pulmonary hypertension in left heart disease, pulmonary hypertension in lung disease and/or hypoxia, and pulmonary hypertension in chronic thromboembolism.

4. The medical active ingredient for use according to any one of the preceding claims, wherein the solvate is a hydrate.

5. The medical active ingredient for use according to any one of the preceding claims, wherein the pharmaceutically acceptable salt comprises a cation selected from the group comprising: sodium, potassium, calcium and magnesium.

6. The medical active ingredient for use according to any one of claims 1 to 4, wherein the pharmaceutically acceptable salt comprises an anion selected from the group comprising: acetate, lactate, citrate, oxalate, glutarate, malate, tartrate, bitartrate, fumarate, mandelate, maleate, succinate, benzoate, chloride, hydrogen carbonate and phthalate.

7. A pharmaceutical formulation for use in the therapeutic treatment and/or prevention of pulmonary hypertension, comprising the medical active ingredient according to any one of the preceding claims and a pharmaceutically acceptable excipient.

8. The pharmaceutical formulation for use in the therapeutic treatment and/or prevention according to claim 7, wherein the pharmaceutically acceptable excipient is selected from the group comprising lactose, cellulose, starch, sucrose, paraffin, hard fat, polyethylene glycol (PEG), polyethylene oxide (PEO), water, ethanol, electrolyte solution.

9. The pharmaceutical formulation for use in the therapeutic treatment and/or prevention according to claim 7 or 8, wherein the pharmaceutical formulation comprises the medical active ingredient in a dosage of 10 to 500 mg/kg body weight, preferably 50 to 200 mg/kg body weight, more preferably 70 to 80 mg/kg body weight.

10. The pharmaceutical formulation for use in the therapeutic treatment and/or prevention according to any one of claims 7 to 9, wherein the pharmaceutical formulation is provided in a dosage form selected from the group comprising tablets, effervescent tablets, pills, hard capsules, soft capsules, suppositories, injection solutions, powder, granulate, inhalation spray, spray and ointments.

## Revendications

1. Principe actif médical selon la formule (I) : 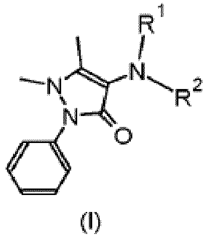
ses sels pharmaceutiquement acceptables et solvates, solvates des sels pharmaceutiquement acceptables du principe actif mentionné, destiné à être utilisé dans le traitement thérapeutique et/ou dans la prévention de l'hypertonie pulmonaire,
dans lequel R¹ et R² sont choisis indépendamment l'un de l'autre parmi le groupe comprenant de l'hydrogène, un groupe alkyle en C1-C4 substitué et non substitué, et
dans lequel le groupe alkyle en C1-C4 substitué dispose de 1 à 4 atomes de carbone et ses atomes de carbone sont substitués facilement ou à maintes reprises par du deutérium ou du fluor ou du -SO₃H.

2. Principe actif médical destiné à être utilisé dans le traitement thérapeutique et/ou dans la prévention de l'hypertonie pulmonaire selon la revendication 1, dans lequel le principe actif médical comprend un composé choisi parmi le groupe comprenant : 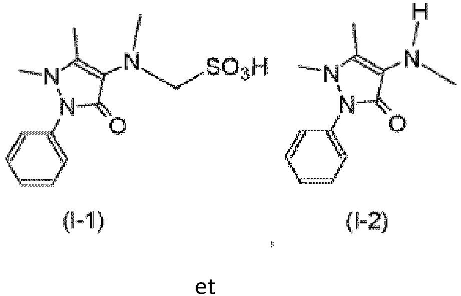 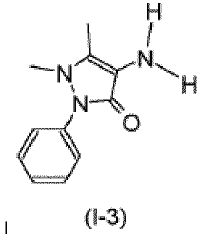 et ses sels pharmaceutiquement acceptables, des solvates.

3. Principe actif destiné à être utilisé dans le traitement thérapeutique et/ou dans la prévention de l'hypertonie pulmonaire selon la revendication 1 ou 2, dans lequel l'hypertonie pulmonaire comprend une hypertonie artérielle pulmonaire, une hypertonie pulmonaire dans le cas d'affections du coeur gauche, une hypertonie pulmonaire dans le cas d'affections des poumons et/ou une hypoxie et une hypertonie pulmonaire dans le cas d'une maladie thromboembolique chronique.

4. Principe actif médical destiné à être utilisé selon l'une quelconque des revendications précédentes, dans lequel le solvate est un hydrate.

5. Principe actif médical destiné à être utilisé selon l'une quelconque des revendications précédentes, dans lequel le sel pharmaceutiquement acceptable comprend un cation, qui est choisi parmi le groupe comprenant : du sodium, du potassium, de calcium et du magnésium.

6. Principe actif médical destiné à être utilisé selon l'une quelconque des revendications 1 à 4, dans lequel le sel pharmaceutiquement acceptable comprend un anion, qui est choisi parmi le groupe comprenant : de l'acétate, du lactate, du citrate, de l'oxalate, du glutarate, du malate, du tartrate, du bitartrate, du fumarate, du mandélate, du maléate, du succinate, du benzoate, du chlorure, de l'hydrogénocarbonate et du phtalate.

7. Formulation pharmaceutique destinée à être utilisée dans le traitement thérapeutique et/ou dans la prévention de l'hypertonie pulmonaire, comprenant le principe actif médical selon l'une quelconque des revendications précédentes et un adjuvant pharmaceutiquement acceptable.

8. Formulation pharmaceutique destinée à être utilisée dans le traitement thérapeutique et/ou dans la prévention selon la revendication 7, dans laquelle l'adjuvant pharmaceutiquement acceptable est choisi parmi le groupe comprenant du lactose, de la cellulose, de l'amidon, du saccharose, de la paraffine, de la matière grasse dure, du polyéthylèneglycol (PEG), du polyoxyde d'éthylène (PEO), de l'eau, de l'éthanol, une solution électrolytique.

9. Formulation pharmaceutique destinée à être utilisée dans le traitement thérapeutique et/ou dans la prévention selon la revendication 7 ou 8, dans laquelle la formulation pharmaceutique comprend le principe actif médical en un dosage de 10 à 500 mg/kg de poids corporel, de préférence 50 à 200 mg/kg de poids corporel, de manière particulièrement préférée de 70 à 80 mg/kg de poids corporel.

10. Formulation pharmaceutique destinée à être utilisée dans le traitement thérapeutique et/ou dans la prévention selon l'une quelconque des revendications 7 à 9, dans laquelle la formulation pharmaceutique est fournie en une forme posologique choisie parmi le groupe comprenant des comprimés, des comprimés effervescents, des pilules, des capsules dures, des capsules molles, des suppositoires, des solutions d'injection, de la poudre, des granulés, un spray d'inhalation, un spray et des pommades.
